(19) Europäisches Patentamt
European Patent Office
Office européen des brevets

(11) **EP 2 408 731 B1**

(12) **EUROPÄISCHE PATENTSCHRIFT**

(45) Veröffentlichungstag und Bekanntmachung des Hinweises auf die Patenterteilung:
**01.04.2015 Patentblatt 2015/14**

(21) Anmeldenummer: **10704837.3**

(22) Anmeldetag: **23.02.2010**

(51) Int Cl.:
*C07C 45/74* (2006.01)        *C07C 47/21* (2006.01)
*B01J 19/24* (2006.01)

(86) Internationale Anmeldenummer:
**PCT/EP2010/052271**

(87) Internationale Veröffentlichungsnummer:
**WO 2010/105892 (23.09.2010 Gazette 2010/38)**

(54) **VERFAHREN ZUR HERSTELLUNG VON A,B-UNGESÄTTIGTEN C10-ALDEHYDEN**

METHOD FOR PRODUCING A,B-UNSATURATED C10-ALDEHYDES

PROCÉDÉ DE PRÉPARATION D'ALDÉHYDE EN C10 A,B-INSATURÉ

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **17.03.2009 DE 102009001594**

(43) Veröffentlichungstag der Anmeldung:
**25.01.2012 Patentblatt 2012/04**

(73) Patentinhaber: **Evonik Degussa GmbH**
**45128 Essen (DE)**

(72) Erfinder:
• **KAIZIK, Alfred**
  **45772 Marl (DE)**
• **FRIDAG, Dirk**
  **45721 Haltern am See (DE)**
• **LÜKEN, Hans-Gerd**
  **45770 Marl (DE)**
• **BÜSCHKEN, Wilfried**
  **45721 Haltern am See (DE)**

(56) Entgegenhaltungen:
**EP-A1- 1 103 538      EP-A2- 1 106 596**

**Beschreibung**

Die vorliegende Erfindung betrifft die Herstellung von $\alpha,\beta$-ungesättigten $C_{10}$-Aldehyden, insbesondere von 2-Propylhept-2-enal, durch Aldolkondensation von $C_5$-Aldehyden, insbesondere n-Pentanal mit Hilfe eines kontinuierlichen Verfahrens nach dem Oberbegriff des Anspruch 1.

Ein Verfahren dieser Gattung ist aus der DE 199 57 522 bekannt.

Aus $\alpha,\beta$-ungesättigten $C_{10}$-Aldehyden werden durch Totalhydrierung Decanole gewonnen, die begehrte Weichmacheralkohole sind. Decenole sind Zwischenstufen für die Herstellung von Decansäuren, die zur Erzeugung von Perestern, Detergenzien, Weichmacher und Schmiermitteln eingesetzt werden können.

In DE 101 08 474, DE 101 08 475, DE 101 08 476 und DE 102 25 282 wird u. a. erwähnt, das $C_5$-Aldehyde, insbesondere n-Pentanal, zu $\alpha,\beta$-ungesättigten $C_{10}$-Aldehyden umgesetzt werden können. Genaue Bedingungen zur Durchführung der Aldolkondensation werden nicht offenbart. Es wird lediglich darauf hingewiesen, dass $\alpha,\beta$-ungesättigte $C_{10}$-Aldehyde aus aliphatischen $C_5$-Aldehyden analog wie Octenal, 2-Ethylhex-2-enal, aus n-Butanal (Butyraldehyd) hergestellt werden können.

Bei der Umsetzung von aliphatischen $C_5$-Aldehyden zu $\alpha,\beta$-ungesättigten $C_{10}$-Aldehyden durch Aldolkondensation werden in der praktizierten Technik vorzugsweise basische Homogenkatalysatoren in Form von Laugen, insbesondere als wässrige NaOH, eingesetzt. In einem ersten Reaktionsschritt der Aldolisierung entsteht ein $C_{10}$-Hydroxyaldehyd ($C_{10}$-Aldol), aus dem in einem zweiten Reaktionsschritt durch Abspaltung von Wasser der ungesättigte $C_{10}$-Aldehyd (Decenal) gebildet wird. Die Reaktion läuft unter Beteiligung von zwei Phasen (organische Aldehyd-Phase, wässrige Katalysator-Phase) ab, die praktisch nicht mischbar sind. Die Erzielung von hohen Umsätzen und Selektivitäten setzt daher voraus, dass die beiden ineinander nicht mischbaren Flüssigphasen während der Reaktion miteinander in innigen Kontakt gebracht werden, um die Hemmung des Stoffübergangs zwischen den Phasen zu überwinden. Durch geeignete verfahrenstechnische Maßnahmen muss daher eine möglichst große Stoffübergangsfläche zwischen beiden Phasen erzeugt werden.

Nach dem Stand der Technik wird der Stoffübergang zwischen der organischen Pentanal-haltigen Phase und der wässrige Katalysatorphase bei dem Einsatz von Rührkessel durch intensives Rühren und bei der Verwendung von Rohrreaktoren durch turbulente Strömung gewährleistet.

In WO 93/20034 wird die Aldolkondensation von aliphatischen $C_5$-Aldehyden zu $\alpha,\beta$-ungesättigten $C_{10}$-Aldehyden in einem Rührkessel beschrieben. Als Katalysator wird ca. 2%ige Natronlauge bei einer Reaktionstemperatur von 120 °C verwendet. Die Reaktion wird kontinuierlich in einem Rührkessel bei einem Phasenverhältnis von organischer Phase zu Natronlauge im Bereich von 0,5 zu 1 bis 5 zu 1 durchgeführt. Um im geraden Durchgang einen Umsatz von 97 % zu erreichen ist eine Verweilzeit von 50 Minuten erforderlich. Bevorzugt wird bei einer Verweilzeit von 30 Minuten ein Umsatz von ca. 70 % angestrebt. Aus dem Reaktionsprodukt werden die nicht umgesetzten $C_5$-Aldehyde destillativ abgetrennt und in den Reaktor zurückgefahren.

In EP 1 103 538 wird ein Verfahren zur Aldolkondensation unter adiabatischer Reaktionsführung und anschließender Aufarbeitung mittels Kurzwegdestillation beschrieben.

In DE 199 57 522 erfolgt die Umsetzung von aliphatischen $C_5$-Aldehyden zu $\alpha,\beta$-ungesättigten $C_{10}$-Aldehyden in Gegenwart von Natronlauge in einem Rohrreaktor, in dem die organische Phase in der Natronlauge dispergiert ist und der laut Beispielen mit einem Belastungsfaktor größer 9,92 betrieben wird. Dabei ist der Belastungsfaktor B wie folgt definiert:

$$B = PD/PS$$

PD [Pa/m] ist ein längenbezogener Druckverlust über den Reaktor unter Betriebsbedingungen und PS [Pa/m] eine Rechengröße mit der Einheit eines längenbezogenes Druckes, definiert als Verhältnis von Massenstrom M [kg/s] aller Komponenten im Reaktor zum Volumenstrom V [m³/s] aller Komponenten unter Betriebsbedingungen, multipliziert mit g = 9,81 m/s².

Mit diesem Verfahren können Pentanal-Umsätze von über 96 % in hohen Raum-Zeit-Ausbeuten bei einem Natronlauge/Edukt-Verhältnis von ca. 100 zu 1 erreicht werden.

Die Verfahren nach WO 93/20034 und DE 199 57 522 weisen den Nachteil auf, dass für ihre Durchführung ein sehr hoher Energieaufwand notwendig ist. Dieser ist bei der Rührkesselreaktion in WO 93/20034 durch die Antriebsleistung der Rührorgane und bei der aus DE 199 57 522 bekannten Rohrreaktion durch die Strömungsverluste durch die Turbulenzen bedingt.

Der vorliegenden Erfindung liegt daher die Aufgabe zu Grunde, ein Verfahren der eingangs genannten Gattung so weiterzubilden, dass seine Durchführung bei hohen Produktausbeuten weniger Energieeinsatz bedarf.

Gelöst wird diese Aufgabe dadurch, dass die aliphatischen $C_5$-Aldehyde und/oder die $\alpha,\beta$-ungesättigten $C_{10}$-Al-

dehyde als Tropfen in der wässrigen Base dispergiert werden, wobei der durchschnittliche Sauter-Durchmesser der Tropfen zwischen 0,2 mm und 1,54 mm liegt.

Gegenstand der Erfindung ist mithin ein Verfahren zur kontinuierlichen Herstellung von $\alpha,\beta$-ungesättigten $C_{10}$-Aldehyden aus aliphatischen $C_5$-Aldehyden, welches die folgenden Schritte umfasst:

a) Aldolkondensation von aliphatischen $C_5$-Aldehyden zu $\alpha,\beta$-ungesättigten $C_{10}$-Aldehyden in Gegenwart einer wässrigen Base in einem Rohrreaktor;

b) Phasentrennung des Austrags des Rohrreaktors in eine wässrige Katalysatorphase und eine organische Produktphase;

c) Auftrennung der organischen Produktphase in $\alpha,\beta$-ungesättigte $C_{10}$-Aldehyde, aliphatische $C_5$-Aldehyde und Nebenprodukte;

d) Ausschleusung eines Teils der wässrigen Katalysatorphase zur Entfernung des Reaktionswassers und Ergänzung mit Frischlauge und anschließender Rückführung in den Rohrreaktor;

wobei die aliphatischen $C_5$-Aldehyde und/oder die $\alpha,\beta$-ungesättigten $C_{10}$-Aldehyde als Tropfen in der wässrigen Base dispergiert werden; und wobei der durchschnittliche Sauter-Durchmesser der Tropfen zwischen 0,2 mm und 1,54 mm liegt.

Die Erfindung beruht unter anderem auf der Grunderkenntnis, dass ein Stoffaustausch zwischen den Phasen der Reaktion besonders effektiv erfolgt, wenn das Edukt bzw. das Edukt/Produkt-Gemisch in der wässrigen Base unter Einhaltung der angegebenen Tropfengröße dispergiert wird. Überraschenderweise zeigte sich, dass diese Tropfengröße sich unter sehr geringem Energieeinsatz mit Mischmodulen einstellen lässt und zudem einen hohen Stoffaustausch und daher eine hohe Produktausbeute gewährleistet.

Mischmodule im Sinne der Erfindung sind passive Einbauten im Strömungsweg, welche die erfindungsgemäße Dispersion bewerkstelligen.

Der erfindungsgemäße Effekt kann wie folgt erläutert bzw. gedeutet werden:

Die Aldolkondensation von aliphatischen $C_5$-Aldehyden wird als Zweiphasenreaktion maßgebend durch die Phasengrenzfläche zwischen der dispersen und der kontinuierlichen Phase bestimmt. Die organische $C_5$-Aldehyd-Phase ist die disperse Phase, die wässrige NaOH die kontinuierliche Phase. Die Größe der Phasengrenzfläche hängt von dem Tropfendurchmesser und dem Anteil der dispersen Phase $\varphi$ ab. Für die spezifische Phasengrenzfläche a gilt:

$$a = 6^* \varphi / d_s \ [1/m]$$

wobei $d_s$, den über alle Tropfen summierten Sauter-Durchmesser darstellt.

Der Sauter-Durchmesser $d_s$ beschreibt den mittleren Tropfendurchmesser der dispersen $C_5$-Aldehyd-Phase. Je feiner die Tropfenverteilung (kleinerer Sauter-Durchmesser) ist, desto größer ist die Austauschfläche.

Für die Berechnung der spezifischen Phasengrenzfläche ist der Sauter-Durchmesser erforderlich, welcher über eigene experimentelle Ergebnisse oder solchen aus der Literatur bestimmt werden muss [Coulaloglou C.A. AICHE Journal, 22, No.2 (1976), pp. 289-295 ,10 und R. K. Thakur at al. Trans IChemE, Vol 81; 2003, pp. 787-826].

Für gerührte Flüssigkeit/Flüssigkeit-Systeme wurde von mehreren Autoren anhand der Experimente eine Reihe von Berechnungsgleichungen für die Bestimmung des Sauter-Durchmessers aufgestellt. Der überwiegende Teil, der aus der Literatur bekannten Gleichungen, lässt sich durch folgende Gleichung zusammenpassen:

$$d_S / d_R = C_1 {}^* We^{-0.6} (1 + C_2 {}^* \varphi)$$

Hierbei sind $C_1$ und $C_2$ systemabhängige Konstanten, die zwischen 0,027 und 0,081 für $C_1$ und zwischen 0,97 und 23,3 für $C_2$ liegen. We ist die Weber-Zahl und $d_R$ der Durchmesser des Rührers. Die We-Zahl stellt das Verhältnis aus Dispergierkraft und Grenzkraft dar.

$$We = (1 / \sigma) {}^* \rho {}^* d_R^3 {}^* n^2$$

Hierbei sind n die Rührer-Drehzahl, $\rho$ die Dichte und $\sigma$ die Oberflächenspannung.

Zur Berechnung des mittleren Tropfendurchmessers bei der Verwendung von Rührkesseln mit eingebautem Rührer kann folgende Gleichung benutzt werden:

$$d_S = d_R * We^{-0,6}(1 + 4,47*\varphi)*0,081$$

Ähnliche Korrelationen sind jedem Fachmann aus der Literatur auch für die Dispergierung von Flüssig/Flüssig-Systemen bei der Verwendung von statistischen Mischern, wie z. B. Sulzer- oder Kenicks-Mischer, bekannt.

Für die Dispergierung von niederviskosen Flüssigkeiten werden zum Beispiel statische Mischer vom SMV-Typ der Firma Sulzer empfohlen.

Für die statischen SMV-Mischer kann der mittlere Tropfendurchmesser $d_S$ nach Sauter nach folgender Beziehung (Streiff F.; Recent Prog.Genie Proc.11.No.51 (1997) p.307) bestimmt werden:

$$d_S / d_h = 0,21*We^{-0,5} Re^{0,15}$$

wobei $d_h$ den hydraulischen Durchmesser der Mischelementkanäle darstellt.

Die Weber- und die Reynolds-Zahl werden wie folgt definiert:

$$We = \rho * d_h * u^2 / (\sigma * \varepsilon^2)$$

und

$$Re = \rho * d_h * u / (\eta * \varepsilon)$$

wobei u die Geschwindigkeit der flüssigen Phase (m/s) bezogen auf das Leerrohr, $\varepsilon$ das relative Lückenvolumen des Mischers und n die dynamische Viskosität (Pa*s) der kontinuierlichen Phase darstellen.

Der Druckverlust in einem Strömungsrohr mit einem statischen Mischer kann für Mehrphasenströmung von Flüssigkeiten näherungsweise gleich wie für die Einphasenströmung berechnet werden:

$$\Delta p = \xi * \rho * L * u^2 / (2 * d_h)$$

wobei $\xi$ den von der Geometrie abhängigen Widerstandsbeiwert bedeutet. Eine Auflistung der Widerstandsbeiwerte für statische Mischer unterschiedlicher Geometrie ist in der Fachliteratur (R.K.Thakur at all. Trans IChemE, Vol 81, 2003, pp. 787 - 826) zu finden.

Mit der Kenntnis des längenbezogenes Druckes PD (PD=$\Delta$p/L) und der PS-Rechengröße kann, wie in DE 199 57 522 offenbart, der Belastungsparameter B berechnet werden.

Gemeinsames Merkmal der statischen Mischer ist, dass ihr hydrodynamisches Verhalten eingehend untersucht wurde und somit bekannt ist. Diese Tatsache kann als Schlüssel für die sichere Übertragung von Versuchsergebnissen (scale up) auf die Großanlage betrachtet werden. Das Scale-up Risiko bei statischen Mischern ist wegen der streng definierten Geometrie und Strömungsführung sehr gering. Als Grundlage für die Modellübertragung sind zwei Voraussetzungen unabdingbar: gleiche Stoffwerte und gleiche geometrische Ähnlichkeit. Das Übertragungskriterium setzt einen gleichen volumenbezogenen Energieeintrag und einen gleichen Strömungszustand voraus. Der Energieeintrag beim Einsatz statischer Mischer kann gemäß F. Streiff (Recents Progres en Genie des Procedes 11. No.51, 1997) nach folgender Gleichung berechet werden:

$$E_d = (\Delta p * V) / (V_m * \rho) = (Ne * u^3) / (d_R * \varepsilon)$$

Wobei $V_m$ das Mischervolumen in m$^3$, V der Volumenstrom in m$^3$/s und $d_R$ der Rohrdurchmesser bedeuten. Ne ist die Newton-Zahl, eine abgewandelte Widerstandzahl, die von Fa. Sulzer für die Berechnung des Druckabfalls verwendet wird. Die Ne-Zahlen für die einzelnen Mischertypen sind bekannt und in Tabellenform katalogisiert.

Für die Abschätzung des Energieeintrages des Rührers wird zuerst seine Leistung P aus der bekannten Beziehung (Leistungsgleichung) errechnet:

$$P = Ne * \rho * n^3 * d_R^5$$

Wobei Ne ist die Newton-Zahl des Rührwerkes ist. Sie hängt von der Re-Zahl ab. Die Ne-Zahl kann aus den Ne-Re-Diagrammen für unterschiedliche Rührsysteme entnommen werden. Solche Diagramme sind in der Fachliteratur an mehreren Stellen, z. B. in "Rührtechnik -Theorie und Praxis", Springer-Verlag, 1999 von M. Zlokarnik, zu finden. Ist die Rührerleistung und das Reaktionsvolumen $V_R$ bekannt, so kann aus dem Quotient P zu $V_R$ der volumenbezogenen Energieeintrag berechnet werden.

Durch die oben genannten Gleichungen alleine kann die Ausbeute und die Selektivität der $\alpha,\beta$-ungesättigten $C_{10}$-Aldehyde jedoch nicht berechnet werden. Es fehlen weitere Einflussgrößen, wie beispielsweise Temperatur, Phasenverhältnis, Verweilzeit oder Basenzusammensetzung, die genauso wichtig sind.

Hierzu lehrt die Erfindung Folgendes:

Es wurde gefunden, dass aliphatische $C_5$-Aldehyde zu über 96 % zu $\alpha,\beta$-ungesättigten $C_{10}$-Aldehyden umgesetzt werden, wenn die Reaktion in einem Rohrreaktor, der mindestens ein Mischmodul enthält, das das Edukt $C_5$-Aldehyd in Tröpfchen mit einem durchschnittlichen Durchmesser (Sauter-Durchmesser) von 0,2 mm bis 1.54 mm in der kontinuierlichen Katalysatorphase (Prozesslauge) dispergiert, die aus Natronlauge und Natriumsalzen von Carbonsäuren besteht und einen Natriumgehalt von 0,6 bis 1,75 Massen-% sowie einen pH-Wert im Bereich von 12,5 bis 13,5 aufweist.

Weitere vorteilhafte Ausführungsformen der Erfindung ergeben sich aus den Unteransprüchen sowie aus der folgenden Beschreibung.

Die Vorteile des erfindungsgemäßen Verfahrens liegen in der hohen Produktausbeute im geraden Durchgang und im geringen spezifischen Energieverbrauch, wobei der Energieverbrauch durch die Pumpenleistung bestimmt wird; je höher die Pumpenleistung, umso höher der Druckverlust und damit verbunden der spezifische Energieeintrag.

Einsatzstoffe

Im erfindungsgemäßen Verfahren können 3-Methylbutanal, n-Pentanal und ihre Gemische eingesetzt werden. Diese Gemische können bis zu 10 Massen-%, vorzugsweise weniger als 5 Massen-% 2-Methylbutanal aufweisen. Ein bevorzugtes Edukt ist n-Pentanal, das weniger als 10 Massen-%, insbesondere weniger als 5 Massen-% 2-Methylbutanal und weniger als 3 Massen-% 3-Methylbutanal enthält. Ganz besonders bevorzugt wird ein $C_5$-Aldehydgemisch eingesetzt, das einen n-Pentanalgehalt von mindestens 95 % aufweist.

Es sei jedoch darauf hingewiesen, dass auch $C_5$-Aldehydgemische, die eine andere Zusammensetzung als die oben genannten Gemische aufweisen, im erfindungsgemäßen Verfahren eingesetzt werden können. Beispielsweise könnten die Einsatzstoffe geringe Mengen an Pentanolen enthalten.

Ein n-Pentanal reiches $C_5$-Aldehydgemisch kann durch Hydroformylierung von 1-Buten, 2-Buten oder Gemische davon, die jeweils nur geringe Anteile von Isobuten aufweisen, erhalten werden. Ein Verfahren zur Herstellung von n-Pentanal aus 2-Butenen wird beispielsweise in DE 10 2008 002187.3 beschrieben.

Zur Bildung der Prozesslauge wird im erfindungsgemäßen Verfahren Natronlauge verwendet. Die Natronlauge bildet zusammen mit der Rücklauge die Prozesslauge. Die Rücklauge enthält neben Natriumhydroxid Natriumsalze von Carbonsäuren, hauptsächlich von Pentansäuren. Die Carbonsäuresalze sind im Wesentlichen durch Cannizzaro-Reaktion entstanden.

Im erfindungsgemäßen Verfahren liegt der Natriumgehalt der Prozesslauge am Reaktoreingang bei 0,60 bis 1,75 Massen-%, insbesondere bei 1,1 bis 1,20 Massen-%. Zur Einstellung der Natriumkonzentration der Prozesslauge wird in die Rücklauge Natronlauge mit einer Konzentration größer als 2,5 Massen-% eingespeist. Um wenig Wasser in das Reaktionssystem einzubringen, wird bevorzugt Natronlauge mit höherer Konzentration verwendet. Im erfindungsgemäßen Verfahren wird bevorzugt Natronlauge im Konzentrationsbereich von 5 bis 30 Massen-%, beispielsweise mit 10 Massen-% verwendet.

**Rohrreaktor mit statischen Mischern**

Das erfindungsgemäße Verfahren wird in einem Rohrreaktor durchgeführt, der mindestens ein Mischmodul, bevorzugt mehrere Mischmodule aufweist. Insbesondere beträgt die Anzahl der Mischmodule 1 bis 30, ganz besonders 10 bis 20.

Ein Mischmodul versteht sich als statischer Mischer, also als ein passives Bauteil, welches keinen direkten Eigenenergiebedarf hat.

Der Rohrreaktor besteht aus einem Rohr, das bevorzugt senkrecht ausgerichtet ist. Es kann von unten nach oben oder umgekehrt durchströmt werden. Ein technischer Reaktor kann auch aus mehreren parallel angeordneten Rohren bestehen, die mit U-Rohren miteinander verbunden sind.

Vorzugsweise befindet sich am Reaktoreingang ein Mischmodul. Zwischen den Mischmodulen befinden sich Leerräume. Der Volumenanteil außerhalb des/der Mischmodul(e) am Gesamtvolumen des Reaktors beträgt 20 bis 80 %, insbesondere 30 bis 60 %. Die Mischmodule können gleiche oder unterschiedliche Abstände voneinander aufweisen. Vorzugsweise nimmt der Abstand zwischen den Mischermodulen in der Fließrichtung ab. Die Abstände der Mischermodule zueinander betragen in Abhängigkeit von der vorgesehenen Leerrohrgeschwindigkeit, dem Phasenverhältnis zwischen Edukt- und Katalysatorphase, Reaktionsfortschritt und dem Mischertyp das 0,2 bis fünffache der Mischmodullänge, insbesondere das 0,5 bis zweifache der Mischmodullänge.

Das Mischmodul besteht aus einem statischen Mischer oder aus einer Anordnung von mehreren, bevorzugt zwei statischen Mischern.

Besteht das Mischermodul aus zwei gleichen statischen Mischern sind diese bevorzugt verdreht um die Längsachse des Reaktors angeordnet, insbesondere verdreht um einen Winkel von 45° bis zu 90°. Vorzugsweise werden Mischelemente in dem Mischermodul mit einem Abstand von zwei Rohrdurchmessern angeordnet.

Ein Mischmodul kann auch aus statischen Mischern unterschiedlicher Bauart bestehen. Es kann vorteilhaft sein, dass bei einem Mischermodul, bestehend aus zwei statischen Mischern, der erste einen geringeren hydraulischen Durchmesser aufweist als der zweite. Dabei werden im ersten statischen Mischer möglichst kleine Tröpfchen erzeugt und im zweiten statischen Mischer durch Wahl eines größeren hydraulischen Durchmessers die Koaleszenz des Tropfenschwarms verhindert.

Der hydraulische Durchmesser der Mischelemente der Mischermodule nimmt mit der Fließrichtung vorzugsweise ab.

Im Reaktor können die Mischermodule gleich oder unterschiedlich sein, d. h. sie können derselben oder unterschiedlicher Bauart sein.

Als Mischelemente können alle statischen Mischer verwendet werden, die unter den vorgesehenen Reaktionsbedingungen in der Lage sind, die organische Phase in der Katalysatorphase in Tröpfchen mit einem durchschnittlichen Sauter-Durchmesser im Bereich von 0,2 bis 1,54 mm zu dispergieren.

Als statische Mischer können im erfindungsgemäßen Verfahren Mischelemente eingesetzt werden, die für die Dispergierung von zwei nicht mischbaren, niederviskosen Flüssigkeiten geeignet sind, wie sie kommerziell erhältlich sind.

## Reaktionsbedingungen

Erfindungsgemäß wird die Aldolkondensation der aliphatischen $C_5$-Aldehyde im Temperaturbereich von 100 bis 150 °C, insbesondere im Bereich von 110 bis 140 °C, ganz besonders im Bereich von 120 bis 140 °C durchgeführt.

Die Umsetzung kann in den genannten Temperaturbereichen isotherm, adiabatisch oder polytrop durchgeführt werden. Beispielsweise kann am Reaktoreingang eine Temperatur von 120 °C und am Reaktorausgang eine Temperatur von 140 °C vorliegen.

Der Reaktionsdruck im Reaktor liegt mindestens so hoch, dass sowohl die Prozesslauge als auch die organischen Stoffe (Edukt und Produkte) jeweils als flüssige Phase vorliegen. Der Druck liegt im Bereich von 0,2 bis 1,0 MPa, vorzugsweise im Bereich 0,3 bis 0,5 MPa.

Im erfindungsgemäßen Verfahren beträgt das Mengenverhältnis [kg/kg] von Prozesslauge zu Edukt am Reaktoreingang im Bereich von 5 bis 40, insbesondere im Bereich von 10 bis 15.

Die durchschnittliche Leerrohrgeschwindigkeit des Gemisches aus Edukt und Prozesslauge (unter Annahme gleicher Strömungsgeschwindigkeit beider Phasen) in dem technischen Reaktor liegt im Bereich von 0,5 bis 4 m/s, insbesondere im Bereich von 1 bis 2,5 m/s.

Die durchschnittliche Verweilzeit des Reaktionsgemisches im Reaktor beträgt 40 bis 360 s, insbesondere 60 bis 180 s.

Im erfindungsgemäßen Verfahren weisen die in der Prozesslauge dispergierten Tröpfchen der organischen Phase nach Verlassen eines Mischermoduls einen durchschnittlichen Sauterdurchmesser von 0,2 bis 1,54 mm, insbesondere einen von 0,6 bis 1,3 mm auf.

Der Belastungsfaktor liegt im Bereich von 0,2 bis 0,8.

## Aufarbeitung

Der Reaktionsaustrag wird gekühlt und die organische Phase von der Laugenphase getrennt. Im erfindungsgemäßen Verfahren erfolgt die Phasentrennung im Temperaturbereich von 60 bis 130 °C, insbesondere im Bereich von 70 bis 120 °C, ganz besonders im Bereich von 90 bis 110 °C. Die Trennzeiten betragen je nach der gewählten Temperatur 3 bis 10 Minuten. Bei Temperaturen oberhalb von 90 °C liegt die Trennzeit unter 8 Minuten. Als Trennzeit wird die Zeit definiert, nach der die organische Wertproduktphase klar und frei von Spuren an heterogenem Wasser ist.

Zur Abtrennung der schweren, wässrigen Phase von der leichten, organischen Phase können Abscheider eingesetzt werden, die die Phasentrennung mit alleiniger Ausnutzung der Schwerkraft ermöglichen. Diese sogenannten Schwerkraftabscheider können auch mit Einbauten als koaleszenzfördernde Maßnahme zur Erhöhung der Trennleistung ausgeführt werden. Durch die Verwendung von Einbauten wird der Koaleszenz- und Sedimentationsprozess beschleunigt. Als Koaleszenzhilfen können z. B. Platten, Füllkörper, Gewebepackungen oder Faserbettabscheider eingesetzt werden. Schwerkraftabscheider können als liegende Behälter oder als stehende Behälter ausgeführt werden.

Alternativ zu Schwerkraftabscheidern können zur Flüssig-Flüssig-Trennung auch Separatoren nach dem Prinzip der Zentrifugen eingesetzt werden. Durch Fliehkräfte in einer sich drehenden Trommel wird dabei die schwere Phase getrennt.

Um die schwere, wässrige Phase abzutrennen, werden im erfindungsgemäßen Verfahren vorzugsweise Schwerkraftabscheider eingesetzt, bevorzugt Schwerkraftabscheider, ausgeführt als liegende Behälter mit Einbauten.

Ein Teil der abgetrennten Laugenphase wird zur Abtrennung des Reaktionswassers ausgeschleust, der andere Teil wird in den Reaktor zurückgeführt. Mit dem Auschleusestrom werden auch ein Teil der als Nebenprodukte gebildeten Carbonsäuren (als Natriumsalze) und Natriumhydroxid abgetrennt. Dieser Strom kann einer Kläranlage zugeführt werden. Es ist jedoch auch möglich diesen Strom aufzuarbeiten und teilweise in den Prozess zurückzuführen, wie beispielsweise in DE 198 49 922 und DE 198 49 924 beschrieben.

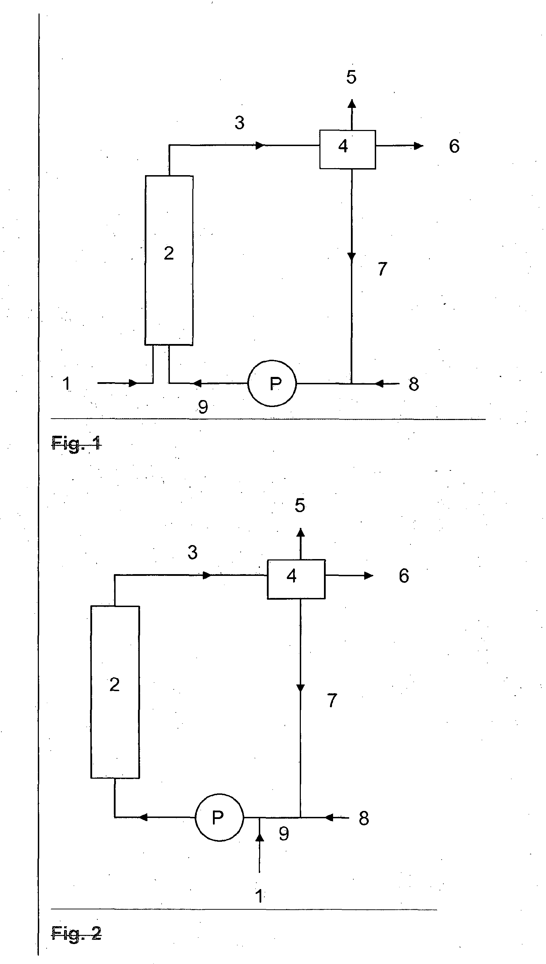

Enthält die organische Phase neben den $\alpha,\beta$-ungesättigten $C_{10}$-Aldehyden und geringen Mengen an nicht umgesetztem Edukt andere Nebenprodukte, wie Carbonsäuresalze, Natriumhydroxid und gelöstes Wasser, so können Basenspuren und ein Teil der Carbonsäuresalze durch eine Wasserwäsche entfernt werden. Der dabei anfallende Wasserextrakt kann zum Ansetzen der Frischlauge verwendet werden (in den Figuren 1 bis 4 nicht eingezeichnet).

Wird reines n-Pentanal als Edukt eingesetzt, enthält die organische Produktphase zwei Decenale, nämlich cis- und trans-2-Propylhept-2-enal.

Enthält das eingesetzte n-Pentanal 2-Methylbutanal und/oder 3-Methylbutarial, kann die organische Phase bis zu acht weitere $\alpha,\beta$-ungesättigte $C_{10}$-Aldehyde enthalten.

Die organische Phase kann destillativ aufgearbeitet werden. Abgetrennte $C_5$-Verbindungen können teilweise in den Reaktor zurückgeführt werden.

Die $\alpha,\beta$-ungesättigten $C_{10}$-Aldehyde können zur Herstellung von Decansäuren (durch Selektivhydrierung und Oxydation) oder zur Herstellung von Decanolen (Totalhydrierung) verwendet werden.

Bei der Herstellung von Decanolen kann optional auch das Rohgemisch hydriert werden und die destillative Auftrennung nach der Hydrierung erfolgen.

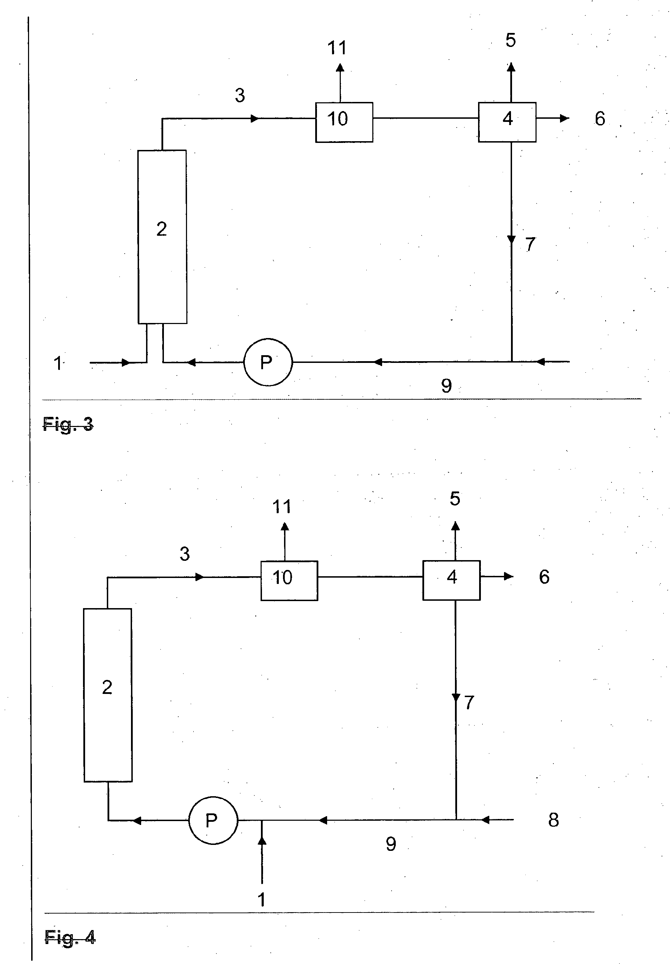

Eine weitere Option der vorliegenden Erfindung ist, das Reaktionsgemisch nach Verlassen des Reaktors und vor der Phasentrennung einer Kurzdestillation zu unterwerfen. Dabei wird das heiße Reaktionsgemisch in einen Behälter entspannt. Als Destillat fällt ein Gemisch aus Wasser und hauptsächlich $C_5$-Verbindungen an, die ganz oder teilweise in den Reaktor zurückgefahren werden können (Trennung des Destillates und Rückführung eines Teils des organischen Destillats in Figur 3 und 4 nicht eingezeichnet). Ein solches Verfahren ist beispielsweise in DE 199 56 410 beschrieben.

## Verfahrensvarianten

Anhand der Figuren 1 bis 4 wird die vorliegende Erfindung nachfolgend näher beschrieben.

Ein Blockschema einer Ausführungsform, in der das erfindungsgemäße Verfahren durchgeführt werden kann, ist in Figur 1 dargestellt. Der Pentanal-Einsatzstrom (1) wird in den Rohrreaktor (2) mit den statischen Mischern eingeleitet. Das den Reaktor verlassende Reaktionsgemisch (3) wird im Trennbehälter (4) in eine organische Phase (5) mit dem Zielprodukt und eine Laugenphase getrennt, von der ein Teil (6) ausgeschleust und der andere Teil (7) zusammen mit Frischlauge (8) als Strom (9) in den Reaktor (2) zurückgeführt wird.

In Figur 1 kann optional der Pentanal Einsatzstrom (1) nach der Umlaufpumpe (P) mit Strom (9) zusammengeführt und in den Reaktor eingeleitet werden.

Figur 2 stellt eine weitere Ausführungsform des erfindungsgemäßen Verfahrens dar. Die Verfahrensvariante nach Figur 2 unterscheidet sich von der Variante nach Figur 1 dadurch, dass der Pentanal-Einsatzstrom (1) vor der Umlaufpumpe in die Anlage eingebracht wird. Durch die intensive Mischung der beiden Phasen findet bereits in der Umlaufpumpe ein Teilumsatz statt, sodass die Verweilzeit im Reaktor verkürzt werden kann. Dieser Effekt ist besonders bei Kreiselpumpen ausgeprägt.

Die Verfahrensvariante nach Figur 3 unterscheidet sich von der Ausführungsform nach Figur 1 dadurch, dass das Reaktionsgemisch (3) vor der Phasentrennung im Behälter (4) einer Kurzdestillation unterworfen wird. Ebenso unterscheidet sich die Verfahrensvariante nach Figur 4 von der der Verfahrensvariante nach Figur 2.

Beispiele

Die folgenden Beispiele sollen die Erfindung erläutern.

Versuchsapparatur / Versuchsauswertung

Die Aldolkondensation von $C_5$-Aldehyden nach dem erfindungsgemäßen Verfahren erfolgte in einer Versuchsanlage, die schematisch der in Figur 1 dargestellten Verfahrensvariante entsprach.

Die kontinuierliche Katalysatorphase 7 und 8 (Natronlauge) wird mit einer Kreiselpumpe P im Kreis geführt. Der wässrigen Katalysatorphase wurde der $C_5$-Aldehyd (n-Pentanal) oder die $C_5$-Aldehydmischung (n-Pentanal/2-Methylbutanal) durch Leitung 1 zugemischt. Die Einspeisung des Aldehydes kann auch vor der Pumpe, wie in Figur 2 dargestellt, erfolgen. Die so erhaltene Mehrphasenmischung aus wässriger Katalysatorphase und organischer Aldehyd-Phase wurde in einem Rohrreaktor 2 aus Edelstahl umgesetzt.

Der nach dem Reaktor anfallende Flüssigkeitsstrom (Produkt- und Katalysatorphase) 3 wurde in einen Phasentrennbehälter 4 geleitet. Hier wurde die wässrige Katalysatorphase (untere Phase) abgetrennt und über Leitung 7 erneut dem Kreislauf zugeführt. Die über ein Wehr des Phasenbehälters gelaufene organische Phase (obere Phase), die das Reaktionsprodukt enthält, kann über Leitung 5 entnommen werden. Das gebildete Reaktionswasser kann über Leitung 6 kontinuierlich ausgeschleust werden. Um die Verluste an Natronlauge durch Ausschleusung des Reaktionswassers zu kompensieren, wird über die Leitung 8 mit einer pH-Wert-geregelten Pumpe kontinuierlich frische 10%ige Natronlauge zudosiert.

Durch die Zudosierung der frischen Natronlauge wurde gewährleistet, dass der pH-Wert der wässrigen Katalysatorphase von 12,70 ± 0,10 während der Versuche konstant gehalten werden konnten.

Die Reaktionswärme wurde über die außerhalb des Reaktors liegenden Wärmetauscher (nicht in Figur 1 angezeichnet) abgeführt.

Für die Durchführung einer vergleichenden nicht erfindungsgemäßen Aldolkondensation von aliphatischen $C_5$-Aldehyden, wie in Beispiel I dargestellt, wurde statt eines mit statischen Mischern befüllten Rohrreaktors ein Rührreaktor verwendet.

Die den Beispielen 1 bis 5 beigefügten Tabellen 1 bis 5 beschreiben im oberen Bereich der Tabellen die Reaktionsbedingungen der $C_5$-Aldehydkondensation. Im unteren Bereich der Tabelle jedes Beispiels ist die Produktzusammensetzung ebenfalls in Massenprozenten der GC-Analyse aufgelistet. Für eine bessere Übersicht wird nicht zwischen den Isomeren der einzelnen $C_{10}$-Aldehyden bzw. $C_{10}$-Hydroxyalkanalen (Aldolen) unterschieden. Diese Werte sind als "2-Propylheptenal" bzw. "$C_{10}$-Aldol" zusammengefasst. Ebenfalls zusammengefasst als "Hochsieder" sind die Nebenprodukte der Aldolisierung, wie Trimere und Tetramere, die aus der Aldolreaktion (Addition und Kondensation) von drei bzw. vier $C_5$-Aldehyden hervorgegangen sind.

Aus den Stoffdaten und den zuvor aufgeführten Gleichungen und Korrelationen wurden der Sauter-Durchmesser und der Energieeintrag für die einzelnen Versuche errechnet. Diese zwei Parameter sind ebenfalls in den Tabellen dargestellt. Die Werte für die Dichte und die Oberflächenspannung können der Fachliteratur entnommen werden, im vorliegenden Falle liegt die Dichte bei ungefähr 1000 kg/m$^3$ und die Oberflächenspannung bei ungefähr 40 * 10$^{-3}$ N/m.

**Beispiel 1 (Vergleichsbeispiel)**

Herstellung von 2-Propylheptenal aus n-Pentanal in einem Rührreaktor:

2-Propylheptenal wurde durch Kondensation von n-Pentanal in einem Rührreaktor in Form einer Extraktionskolonne (Volumen 2,1 Liter) mit 10 Mischkammern, die mit 4-Blatt-Rührer (68,1 mm Durchmesser) angebracht auf einer Rührachse ausgestattet waren, hergestellt. Die kontinuierliche Katalysatorphase (2%ige Natronlauge) wurde mittels einer Kreislaufpumpe im Kreis geführt. Das Edukt n-Valeraldehyd wurde aus einem 100 l Fass (Eduktvorlage) entnommen und kontinuierlich über eine dünne Kapillare vor dem Reaktoreintritt in den NaOH-Kreislauf gepumpt. Das Gemisch aus Produkt- und wässriger Katalysatorphase wurde nach dem Reaktor einem Phasentrennbehälter zugeführt. In dem Phasentrennbehälter wurde die organische Produktphase von der Katalysatorphase abgetrennt. Nach der Abtrennung der Produktphase wurde die wässrige Phase dem NaOH-Kreislauf zugeführt.

Der Katalysatorkreislauf (2,0%ige wässrige NaOH, pH-Wert 12,85) betrug bei allen Versuchen 80 l/h. Das Edukt n-Pentanal wurde mit einem Durchsatz von 8 l/h, entsprechend einem Phasenverhältnis (PV) organische Phase zur wässrige Phase von 1 zu 10, in den NaOH-Kreislauf eingespeist. Das Edukt enthielt neben 98,3 Massen-% n-Pentanal, 1,7 Massen-% Nebenkomponente, darunter 0,2 Massen-% $C_{10}$-Aldole und 0,3 Massen-% Hochsieder.

In Tabelle 1 sind die Ergebnisse der Aldolisierung von n-Pentanal bei 130 °C und 4 bar Druck bei unterschiedlicher Rührdrehzahl (Einheit:Umdrehung pro Minute / Upm) dargestellt. Im kontinuierlichen Betrieb nach 3 Stunden Versuchzeit im stationären Zustand wurden folgende Ergebnisse erhalten:

- Tabelle 1 -

| Reaktionsbedingungen | I | II | III |
|---|---|---|---|
| Rührdrehzahl (Upm) | 500 | 1000 | 2000 |
| Temperatur (°C) | 130 | 130 | 130 |
| PV (l Edukt/l Kat.-Phase) | 1 zu 10 | 1 zu 10 | 1 zu 10 |
| | | | |
| **Produkt-Zusammensetzung** | | | |
| n-Pentanal (Massen-%) | 9,82 | 5,05 | 5,42 |
| 2-Propylheptenal (Massen-%) | 86,25 | 92,14 | 92,25 |
| C10-Aldole (Massen-%) | 0,08 | 0 | 0 |
| | | | |
| **n-Pentanal-Umsatz (%)** | 90 | 94,8 | 94,5 |
| **Sauter-Durchmesser (mm)** | 0,13 | 0,057 | 0,025 |
| **Energieeintrag (W/m³)** | 3806 | 30675 | 245501 |

Wie man der Tabelle 1 entnehmen kann, werden für die Erzielung hoher 2-Propylheptenal-Gehalte > 92 Massen-% im Produktaustrag hohe Rührdrehzahlen größer als 500 Upm erforderlich. Durch höhere Rührdrehzahlen wird offensichtlich die Vermischung der dispersen organischen Phase mit der wässrigen Phasen verbessert. Bei Rührdrehzahlen von 1000 Upm und höher ist kein Einfluss der Drehzahl auf den erzielbaren Umsatz von n-Pentanal festzustellen. Die aus den Korrelationen für den Rührreaktor errechneten Sauter-Durchmesser liegen in Abhängigkeit von Rührdrehzahl zwischen 0,025 und 0,13 mm.

Beispiel 2

Dieses Beispiel beschreibt das erfindungsgemäße Verfahren für die Aldolkondensation von n-Pentanal (n-Valeraldehyd) zu 2-Propylheptenal (1+1-Produkt) und die Co-Aldolisierung von n-Pentanal mit 2-Methylbutanal zu dem Kreuzprodukt(1+2 Produkt) 2-Propyl-4-methyl-hexenal.

Als Reaktor wurde ein 3 m langes DN15-Rohr (Innendurchmesser 17,3 mm) mit einem Gesamtvolumen von 6,8 l verwendet. Rund 50 % des Gesamtvolumens des Rohrreaktors war mit statischen Mischern mit einem hydraulischen Durchmesser von 2 mm und einem relativen Lückenvolumen von 0,80 gefüllt. Die Mischelemente sind aus geriffelten Lamellen aufgebaut, die offene sich kreuzende Kanäle bilden. Die einzelnen Mischelemente mit einem Abstand von einer Mischlänge wurden um 90° zueinander versetzt im Rohreaktor angeordnet. Durch den Einsatz der statischen Mischer sollten die Inhomogenitäten des Reaktionsgemisches über den gesamten Rohrquerschnitt ausgeglichen werden.

Als Edukt für die Versuche wurde ein $C_5$-Aldehydgemisch, bestehend aus 94, 4 Massen-% n-Pentanal und 5,0 Massen-% 2-Methylbutanal sowie 0,6 Massen-% Nebenkomponenten, darunter 0,1 Massen-% Hochsieder, eingesetzt.

Bei einem Kreislauf der Katalysatorphase (2,1 %ige wässrige Natronlauge, pH-Wert 12,98) von 80 l/h wurde kurz vor dem Eintritt in den mit statischen Mischern befüllten Reaktor gemäß Figur 1 Edukt mit einem Durchsatz von 2 l/h bei drei verschiedenen Temperaturen von 110 °C, 120 °C und 130 °C eingeleitet.

Unter den gewählten Reaktionsbedingungen wurde über die Reaktorlänge ein Druckverlust Δp von 0,049 bar ermittelt. Mit den Stoffdaten für das Zweiphasensystem n-Pentanal und wässrige 2%ige Natronlauge wurde unter Einbeziehung der oben aufgeführten Gleichungen ein mittlerer Tropfendurchmesser (Sauter-Durchmesser ds) der dispersen organischen Phase von rd. 0,91 mm errechnet.

Die in Tabelle 2 niedergelegten Ergebnisse wurden nach 3 Stunden im stationären Zustand erzielt:

- Tabelle 2 -

| Reaktionsbedingungen | I | II | III |
|---|---|---|---|
| Temperatur (°C) | 110 | 120 | 130 |
| PV (l Edukt/l Kat.-Phase) | 1 zu 40 | 1 zu 40 | 1 zu 40 |

(fortgesetzt)

| Reaktionsbedingungen | I | II | III |
|---|---|---|---|
| **Produkt-Zusammensetzung** | | | |
| n-Pentanal (Massen-%) | 19,22 | 11,46 | 6,03 |
| 2-Methylbutanal (Massen-%) | 3,48 | 2,56 | 2,79 |
| 2-Propylheptenal (Massen-%) | 73,21 | 81,4 | 86,26 |
| 2-Propyl-4-methyl-hexenal | 3,6 | 3,63 | 4,09 |
| C10-Aldole (Massen-%) | 0,01 | 0 | 0 |
| Hochsieder /Rest (Massen-%) | 0,48 | 0,95 | 0,84 |
| | | | |
| **n-Pentanal-Umsatz** (%) | 79,6 | 87,8 | 93,6 |
| **Sauter-Durchmesser** (mm) | 0,91 | 0,91 | 0,91 |
| **Energieeintrag** ($W/m^3$) | 315 | 315 | 315 |

Wie man der Tabelle 2 entnehmen kann, nimmt der Gehalt an dem Wertprodukt 2-Propylheptenal von 73,21 auf 86,26 Massen-% mit der Temperatursteigerung von 110 auf 130 °C deutlich zu. Demgegenüber fällt die Temperaturabhängigkeit des zweiten Wertproduktes 2-Propyl-4-methyl-hexenal (Kreuzprodukt der n-Pentanal / 2-Methylbutanal-Aldolkondensation) deutlich geringer aus.

Im Vergleich zu Aldolisierungsversuchen im Rührreaktor (siehe Beispiel 1, Spalten II und III der Tabelle 1) werden bei dem Einsatz von statischen Mischern (s. Spalte III der Tabelle 2) vergleichbare Umsätze erzielt, wobei der Energieeintrag und das 97- bis 780-fache geringer ist.

Beispiel 3

Dieses Beispiel beschreibt das erfindungsgemäße Verfahren für die Aldolkondensation von n-Pentanal (n-Valeraldehyd) zu 2-Propylheptenal (1+1-Produkt) und die Co-Aldolisierung von n-Pentanal mit 2-methylbutanol zu dem Kreuzprodukt(1+2 Produkt). 2-Propyl-4-methyl-hexenal. Im Unterschied zum Beispiel 2 wurde als Reaktor nicht ein 3 m langes Rohr, sondern ein 4 m langes Rohr DN15-Rohr (Innendurchmesser 17,3 mm) mit einem Gesamtvolumen von 9;1 l verwendet. Auch in diesem Beispiel wurden rund 50 % des Gesamtvolumens des Rohrreaktors mit statischen Mischern mit einem hydraulischen Durchmesser von 2 mm und einem relativen Lückenvolumen von 0,80 gefüllt.

Als Edukt für die Versuche wurde ein $C_5$-Aldehydgemisch, bestehend aus 93, 7 % Massen-% n-Pentanal und 5,2 Massen-% 2-Methylbutanal sowie 1,1 Massen-% Nebenkomponenten, darunter 0,8 Massen-% $C_{10}$-Aldole und 0,1 Massen-% Hochsieder, eingesetzt. Bei 130 °C und einem Druck von 4 bar wurde bei einem konstant gehaltenen Kreislauf der NaOH-Phase (2,1 %-ige wässrige Natronlauge) von 80 l/h der Durchsatz des Eduktes zwischen 2,2 l/h und 8,4 l/h variiert. Die Dosierung des $C_5$-Aldehydgemisches erfolgte kurz vor dem Eintritt in den mit statischen Mischern befüllten Reaktor gemäß Figur 1. Im kontinuierlichen Betrieb nach 4 Stunden Versuchszeit im stationären Zustand wurden folgende Ergebnisse erhalten:

- Tabelle 3 -

| Reaktionsbedingungen | I | II | III |
|---|---|---|---|
| Temperatur (°C) | 130 | 130 | 130 |
| Edukt -C5-Aldehyde (l/h) | 2,2 | 4,1 | 8,4 |
| PV (I Edukt/I Kat.-Phase) | 1 zu 36 | 1 zu 19,5 | 1 zu 9,5 |
| | | | |
| **Produkt-Zusammensetzung** | | | |
| n-Pentanal (Massen-%) | 3,72 | 5,04 | 11,09 |
| 2-Methylbutanal (Massen-%) | 2,67 | 2,29 | 2,3 |
| 2-Propylheptenal (Massen-%) | 88,51 | 87,59 | 81,2 |
| 2-Propyl-4-methyl-hexenal | 4,6 | 3,8 | 3,55 |
| C10-Aldole (Massen-%) | 0 | 0,18 | 0,87 |
| Hochsieder /Rest (Massen-%) | 0,5 | 0,77 | 0,98 |

(fortgesetzt)

| Produkt-Zusammensetzung | | | |
|---|---|---|---|
| **n-Pentanal-Umsatz** (%) | 96 | 94,6 | 87,2 |
| **Sauter-Durchmesser** (mm) | 0,91 | 0,89 | 0,85 |
| **Energieeintrag** (W/m$^3$) | 318 | 315 | 395 |

Die Erhöhung des Reaktionsvolumens durch Verlängerung des Rohreaktors um einen Meter führte bei gleicher Reaktorbelastung im Vergleich zum Beispiel 2 zu längeren Verweilzeiten und zum Anstieg des Druckverlustes von 0,049 auf 0,066 bar.

Durch die längere Verweilzeit infolge des verlängerten Rohrreaktor konnte der n-Pentanal-Umsatz bei einem Edukt-Durchsatz von rd. 2 l/h von 93,6 % (s. Tabelle 2, Spalte III) auf 96 % (s. Tabelle 3, Spalte I) gesteigert werden.

Wie in der Tabelle 3, Spalte II zu sehen ist, wurden Umsätze > 94 % auch bei einem erhöhten Edukt-Durchsatz von 4,1 l/h erzielt. Die vorliegenden Ergebnisse zeigen, dass die erzielbaren n-Pentanal-Umsätze weniger von dem gewählten Phasenverhältnis, sondern deutlich mehr von der gewählten Verweilzeit der organischen Phase im Reaktor beeinflusst werden. Bei einem Eduktdurchsatz von 8,4 l/h ist die Verweilzeit zu kurz, um einen höheren n-Pentanal-Umsatz als 87,2 % zu erzielen.

Beispiel 4

Dieses Beispiel beschreibt das erfindungsgemäße Verfahren für die Aldolkondensation von n-Pentanal (n-Valeraldehyd) zu 2-Propylheptenal (1+1-Produkt) und die Co-Aldolisierung von n-Pentanal mit 2-Methylbutanal zu dem Kreuzprodukt(1+2 Produkt). 2-Propyl-4-methyl-hexenal. Im Unterschied zum Beispiel 3 wurde das Gesamtvolumens des Rohreaktors (Rohr DN15-Rohr (Innendurchmesser 17,3 mm) mit einem Volumen von 9,1 l Reaktor vollständig mit statischen Mischern mit einem hydraulischen Durchmesser von 2 mm und einem relativen Lückenvolumen von 0,80 gefüllt.

Als Edukt wurde ein $C_5$-Aldehydgemisch, bestehend aus 94, 4 % Massen-% n-Pentanal und 5,0 Massen-% 2-Methylbutanal sowie 0,6 Massen-% Nebenkomponenten, darunter 0,2 Massen-% $C_{10}$-Aldole und 0,1 Massen-% Hochsieder, eingesetzt. Die Zusammensetzung des Edukts war mit der Zusammensetzung des im Beispiel 3 verwendeten $C_5$-Aldehyd-Gemisches vergleichbar.

Bei 130 °C und einem Druck von 4 bar wurde bei einem konstant gehaltenen Kreislauf der NaOH-Phase (2,1 %ige wässrige Natronlauge) von 80 l/h wurden vor dem Eintritt in den Reaktor in den Katalysatorkreislauf 4,1 l/h Edukt gemäß Figur 1 zudosiert.

Im kontinuierlichen Betrieb nach 4 Stunden Versuchszeit im stationären Zustand wurden folgende Ergebnisse erhalten, die in Tabelle 4 Spalte I dargestellt sind. In der Spalte II sind zum Vergleich die Ergebnisse des Versuches aus dem Beispiel 3 in einem Rohrreaktor, dessen Gesamtvolumen zu 50 % mit statischen Mischern belegt wurde, aufgeführt.

- Tabelle 4 -

| Reaktionsbedingungen | I | II |
|---|---|---|
| Volumenanteil der stat. Mischer (%) | 100 | 50 |
| Temperatur (°C) | 130 | 130 |
| Edukt -C5-Aldehyde (l/h) | 4,1 | 4,1 |
| PV (I Edukt/I Kat.-Phase) | 1 zu 19,5 | 1 zu 19,5 |
| | | |
| **Produkt-Zusammensetzung** | | |
| n-Pentanal (Massen-%) | 6,87 | 5,04 |
| 2-Methylbutanal (Massen-%) | 2,32 | 2,29 |
| 2-Propylheptenal (Massen-%) | 86,72 | 87,59 |
| 2-Propyl-4-methyl-hexenal | 3,35 | 3,8 |
| $C_{10}$-Aldole (Massen-%) | 0,05 | 0,18 |
| Hochsieder /Rest (Massen-%) | 0,68 | 0,77 |
| | | |
| **n-Pentanal-Umsatz** (%) | 92,7 | 94,6 |
| **Sauter-Durchmesser** (mm) | 0,89 | 0,89 |

(fortgesetzt)

| Produkt-Zusammensetzung | | |
|---|---|---|
| **Energieeintrag** (W/m3) | 339 | 339 |

Eine vollständige Belegung des Rohrreaktors mit statischen Mischern führte, wie in Tabelle 4 in Spalte I dargestellt, im Vergleich zu 50%iger Befüllung nach dem erfindungsgemäßen Verfahren (Tabelle, Spalte 3) nicht zu einer Erhöhung, sondern zu einem Rückgang des n-Pentanal-Umsatzes.

Beispiel 5

Dieses Beispiel beschreibt das erfindungsgemäße Verfahren für die Aldolkondensation von n-Pentanal (n-Valeraldehyd) zu 2-Propylheptenal (1+1-Produkt) und die Co-Aldolisierung von n-Pentanal mit 2-Methylbutanal zu dem Kreuzprodukt(1+2

Produkt). 2-Propyl-4-methyl-hexenal. Als Reaktor wurde, wie im Beispiel 3, ein 4 m langes DN15-Rohr (Innendurchmesser 17,3 mm) mit einem Gesamtvolumen von 9,1 I verwendet. Von dem Gesamtvolumen des Rohrreaktors war rd. 50 % mit statischen Mischern gefüllt. Im Unterschied zum Beispiel 3 wurde das Edukt $C_5$-Aldehyd-Gemisch nicht vor dem Reaktor, sondern vor der Kreislaufpumpe in den NaOH-Kreislauf gemäß Figur 2 eingespeist.

Als Edukt wurde ein $C_5$-Aldehydgemisch bestehend aus 94,4 % Massen-% n-Pentanal und 5,0 Massen-% 2-Methylbutanal sowie 0,6 Massen-% Nebenkomponenten, darunter 0,2 Massen-% $C_{10}$-Aldole und 0,1 Massen-% Hochsieder. Die Zusammensetzung des Edukts war mit der Zusammensetzung des im Beispiel 3 verwendeten $C_5$-Aldehyd-Gemisches vergleichbar. Bei 130 °C und einem Druck von 4 bar wurden bei einem Kreislauf der wässrigen NaOH-Phase (rd. 2%ige wässrige Natronlauge) von 80 l/h und 40 l/h vor der Kreislaufpumpe in die Katalysatorkreislauf 4,1 l/h Edukt zudosiert.

Durch Herabsetzung des Kreislaufes von 80 auf 40 l/h wird die Strömungshydrodynamik und der mittlerer Tropfendurchmesser (Sauter-Durchmesser) der dispersen Phase verändert. Der auf Basis der zuvor genannten Korrelationen errechnete Sauter-Durchmesser steigt von 0,91 mm auf 1,54 mm.

Im kontinuierlichen Betrieb nach 4 Stunden Versuchzeit im stationären Zustand wurden bei dieser Fahrweise folgende Ergebnisse erhalten, die Tabelle 5 in den Spalten I und II dargestellt sind. In der Spalte III sind zum Vergleich die Ergebnisse des Versuches aus dem Beispiel 3 mit einer Edukt-Zudosierung vor dem Reaktor.

- Tabelle 5 -

| Reaktionsbedingungen | I | I | III |
|---|---|---|---|
| Ort der Edukt-Dosierung | vor Pumpe | vor Pumpe | vor Reaktor |
| Temperatur (°C) | 130 | 130 | 130 |
| NaOH-Kreislauf (l/h) | 40 | 80 | 80 |
| Edukt -C5-Aldehyde (l/h) | 4,1 | 4,1 | 4,1 |
| PV (I Edukt/I Kat.-Phase) | 1 zu 9,7 | 1 zu 19,5 | 1 zu 19,5 |
| | | | |
| **Produkt-Zusammensetzung** | | | |
| n-Pentanal (Massen-%) | 4,53 | 4,77 | 5,04 |
| 2-Methylbutanal (Massen-%) | 2,33 | 2,31 | 2,29 |
| 2-Propylheptenal (Massen-%) | 88,1 | 88,14 | 87,59 |
| 2-Propyl-4-methyl-hexenal | 3,91 | 3,84 | 3,8 |
| C10-Aldole (Massen-%) | 0,11 | 0,17 | 0,18 |
| Hochsieder / Rest (Massen-%) | 1,01 | 0,78 | 0,77 |
| | | | |
| **n-Pentanal-Umsatz** (%) | 95,2 | 94,9 | 94,6 |
| **Sauter-Durchmesser** (mm) | 1,54 | 0,91 | 0,91 |

Die Edukt-Dosierung gemäß Figur 2 führte, wie in Tabelle 5 dargestellt, zu einer Verbesserung der Umsätze. Nach dem vorliegenden erfindungsgemäßen Verfahren können bei der Aldolkondensation von $C_5$-Aldehyden zu $\alpha,\beta$-ungesättigten $C_{10}$-Aldehyden beide aufgeführte Dosierungsvarianten des Eduktes eingesetzt werden.

Beispiel 6: Phasentrennung des Produktes der $C_5$-Aldehyd-Aldolisierung

Dieses Beispiel beschreibt die bevorzugte Phasentrennung des Reaktionsaustrages der Aldolkondensation von n-Pentanal zu 2-Propylheptenal und die Co-Aldolisierung von n-Pentanal mit 2-Methylbutanal zu 2-Propyl-4-methyl-hexenal bei Temperaturen zwischen 70 und 120 °C.

Für die Durchführung der Versuche zur Phasentrennung wurden im Vorfeld aus den laufenden Aldolisierungsversuchen die wässrige Phase und die organische Wertproduktphase nach dem Reaktor entnommen. Für die Versuche zur Phasentrennung wurde ein beheizter 2 l Doppelmantel-Reaktor aus Glas verwendet, der mit einem Rührwerk ausgestattet war. Die Temperatur der Phasentrennung zwischen 40 und 110 °C wurde über einen Thermostaten geregelt. Zuerst wurden die beiden Phasen beim einem Phasenverhältnis (org. Phase/ $H_2O$-Phase) von 1 zu 10 in den Reaktor überführt und der Druck auf 3,5 bar eingestellt, d.h. auf den gleichen Wert wie in der Aldolisierungsanlage. Nachdem die beiden Phasen unter langsamem Rühren auf die Versuchstemperatur gebracht wurden, wurde der Rührer für 2 Minuten bei 500 Umdrehungen pro Minute (U/min) gestartet. Durch das intensive Rühren wurde sichergestellt, dass die beiden Phasen gut ineinander vermischt wurden. Nach Ablauf von 3 Minuten wurde der Rührer gestoppt und die Zeit für die Phasentrennung ermittelt.

Es wurde zu einem die Trennungszeit erfasst, die die beiden Phasen benötigten, um sich vollständig mit einer deutlich sichtbaren Phasengrenzfläche voneinander zu trennen. Zum anderen wurde die Zeit bestimmt, die die Phasen brauchten, um wieder klar.zu sein. Eine klare Wertproduktphase schließt weitgehend die Anwesenheit von feinverteilten, dispersen Wassertropfen in der organischen Phase aus. Bei jeder Temperatureinstellung wurde der Trennungsversuch fünf Mal durchgeführt und der Mittelwert der gewonnenen Ergebnisse gebildet. Die entnommenen organische Phasen wurden nach den Trennungsversuchen mit GC und nasschemisch analysiert.

Mit den nasschemischen Methoden wurden die Wassergehalte (Karl-Fischer) sowie die Natrium-Salzgehalte (titrimetrisch) in den organischen Phasen bestimmt.

In den wässrigen Phasen wurde neben den NaOH- und Na-Salzgehalten zusätzlich der gesamt C-Gehalt ermittelt.

Die für die Trennungsversuche verwendete organische Phase enthielt nach der Analyse rd. 0,79 Massen-% an homogen gelöstem Wasser und keine Natronlaugespuren. Die wässrige NaOH-Phase enthielt neben 2,02 Massen-% NaOH, rd. 0,30 Massen-% Na-Salz und 1,1 Massen-% Gesamt-Kohlenstoff. Die Ergebnisse der Untersuchungen zur Phasentrennung sind in Tabelle 6 zusammengestellt.

- Tabelle 6 -

| Phasentrennung | | | | |
|---|---|---|---|---|
| Temperatur (°C) | 50 | 70 | 90 | 110 |
| Zeit für Phasentrennung (s) | 37 | 25 | 21 | 17 |
| Zeit für Phasen-Aufklärung (min) | 25 | 11 | 8 | 4 |
| | | | | |
| **Analyse / organische Phase** | | | | |
| NaOH-Gehalt (Massen-%) | 0 | 0 | 0 | 0 |
| Wasser (Massen-%) | 1,02 | 0,95 | 0,93 | 1,27 |
| | | | | |
| **Analyse / wässrige Phase** | | | | |
| NaOH-Gehalt (Massen-%) | 2,01 | 2,03 | 2,06 | 2,02 |
| Na-Salz (Massen-%) | 0,33 | 0,34 | 0,31 | 0,34 |
| C-Gesamt (Massen-%) | 1,1 | 1,1 | 1,1 | 1,1 |

Bei dem Na-Salz in Tabelle 6 handelt es sich hauptsächlich um Natriumpentonat.

Mit der Steigerung der Temperatur der Phasentrennung im Bereich von 50 bis 110 ° C wird die Trennung der organischen von der wässrigen Phase verbessert. Dies gilt sowohl für die eigentliche Phasentrennung (Bildung der Phasengrenzfläche), als auch für die Aufklärung der organischen Phase (klare Phase ohne Trübung).

Die Zeit für die Bildung der Phasengrenzflächen ist verhältnismäßig kurz; sie liegt mit 17 bis 37 Sekunden im Sekunden-Bereich. Deutlich langsamer verläuft die Phasenaufklärung, bei der unter anderen die restlichen heterogenen Wassertropfen entfernt werden.

Wie man der Tabelle 6 entnehmen kann, konnte die Zeit für die Phasenaufklärung durch Temperaturerhöhung von 50 auf 70 °C und höher deutlich verkürzt werden.

Nach alledem kann eine besonders bevorzugte Ausführungsform der Erfindung wie folgt angegeben werden:

Ein kontinuierliches Verfahren zur Herstellung von $\alpha,\beta$-ungesättigten $C_{10}$-Aldehyden aus aliphatischen $C_5$-Aldehyden mit diesen Schritten:

a) Aldolkondensation von aliphatischen $C_5$-Aldehyden zu $\alpha,\beta$-ungesättigten $C_{10}$-Aldehyden in Gegenwart einer wässrigen Base in einem Rohrreaktor, der mindestens ein Mischermodul aufweist, welches das Edukt oder Edukt/Produktgemisch als Tröpfchen mit einem durchschnittlichen Sauter-Durchmesser im Bereich von 0,2 bis 1,54 mm in der Prozesslauge dispergiert,

b) Phasentrennung des Reaktoraustrags aus Schritt a) in eine wässrige Katalysatorphase und eine organische Produktphase,

c) Auftrennung der organischen Phase aus Schritt b) in $\alpha,\beta$-ungesättigte $C_{10}$-Aldehyde, aliphatische $C_5$-Aldehyde und Nebenprodukte,

d) Rückführung in den Reaktor eines Teils der wässrigen Phase aus Schritt b) nach Ergänzung mit Frischlauge und teilweiser Ausschleusung des Reaktionswassers, wobei die wässrige Prozesslauge bei einem pH-Wert im Bereich von 12,5 bis 13,5 Natriumhydroxid und Natriumsalze von Carbonsäuren enthält, und

e) Trennung der organischen Produktphase aus Schritt b) von der wässrigen Katalysatorphase bei Temperaturen zwischen 70 und 120 °C.

**Patentansprüche**

1.  Verfahren zur kontinuierlichen Herstellung von $\alpha,\beta$-ungesättigten $C_{10}$-aldehyden aus aliphatischen $C_5$-Aldehyden, welches die folgenden Schritte umfasst:

    a) Aldolkondensation von aliphatischen $C_5$-Aldehyden zu $\alpha,\beta$-ungesättigten $C_{10}$-Aldehyden in Gegenwart einer wässrigen Base in einem Rohrreaktor;
    b) Phasentrennung des Austrags des Rohrreaktors in eine wässrige Katalysatorphase und eine organische Produktphase;
    c) Auftrennung der organischen Produktphase in $\alpha,\beta$-ungesättigte $C_{10}$-Aldehyde, aliphatische $C_5$-Aldehyde und Nebenprodukte;
    d) Ausschleusung eines Teils der wässrigen Katalysatorphase zur Entfernung des Reaktionswassers und Ergänzung mit Frischlauge und anschließender Rückführung in den Rohrreaktor;

    **dadurch gekennzeichnet, dass** die aliphatischen $C_5$-Aldehyde und/oder die $\alpha,\beta$-ungesättigten $C_{10}$-Aldehyde als Tropfen in der wässrigen Base dispergiert werden, wobei der durchschnittliche Sauter-Durchmesser der Tropfen zwischen 0,2 mm und 1,54 mm liegt.

2.  Verfahren nach Anspruch 1, **dadurch gekennzeichnet, dass** der pH-Wert der wässrigen Base im Bereich von 12,5 bis 13,5 liegt, und dass die wässrige Base Natriumhydroxid sowie Natriumsalze von Carbonsäuren enthält.

3.  Verfahren nach Anspruch 1 oder 2, **dadurch gekennzeichnet, dass** die Phasentrennung des Austrags des Rohrreaktors in die wässrige Katalysatorphase und die organische Produktphase bei Temperaturen zwischen 70 °C und 120 °C erfolgt.

4.  Verfahren nach Anspruch 1, 2 oder 3, **dadurch gekennzeichnet, dass** die Reaktionstemperatur im Rohrreaktor im Bereich von 120 °C bis 140 °C liegt.

5.  Verfahren nach einem der Ansprüche 1 bis 4, **dadurch gekennzeichnet, dass** das Verhältnis der Masse von wässriger Base zur Masse der aliphatischen-$C_5$-Aldehyde am Eingang des Rohreaktors im Bereich von 5 bis 20 liegt.

6.  Verfahren nach einem der Ansprüche 1 bis 5, **dadurch gekennzeichnet, dass** die durchschnittliche Leerrohrgeschwindigkeit des Gemisches aus wässriger Base und aliphatischen $C_5$-Aldehyde im Bereich von 0,5 bis 4 m/s, insbesondere im Bereich von 1 bis 2,5 m/s liegt.

**7.** Verfahren nach einem der Ansprüche 1 bis 6, **dadurch gekennzeichnet, dass** die durchschnittliche Verweilzeit des Einsatzgemisches im Rohrreaktor 40 bis 360 Sekunden, insbesondere 60 bis 180 Sekunden beträgt.

**8.** Verfahren nach einem der Ansprüche 1 bis 7, **dadurch gekennzeichnet, dass** ein $C_5$-Aldehydgemisch mit einem n-Pentanalgehalt von mindestens 90 Massen-% eingesetzt wird.

**9.** Verfahren nach einem der Ansprüche 1 bis 8, **dadurch gekennzeichnet, dass** die Dispersion der aliphatischen $C_5$-Aldehyde und/oder der $\alpha,\beta$-ungesättigten $C_{10}$-Aldehyde in der wässrigen Base innerhalb des Rohrreaktors erfolgt.

**10.** Verfahren nach Anspruch 9, **dadurch gekennzeichnet, dass** die Dispersion der aliphatischen $C_5$-Aldehyde und/oder der $\alpha,\beta$-ungesättigten $C_{10}$-Aldehyde in der wässrigen Base innerhalb des Rohrreaktors mit Hilfe mindestens eines im Rohrreaktor eingebauten Mischmoduls erfolgt.

**11.** Verfahren nach Anspruch 10, **dadurch gekennzeichnet, dass** die Dispersion der aliphatischen $C_5$-Aldehyde und/oder der $\alpha,\beta$-ungesättigten $C_{10}$-Aldehyde in der wässrigen Base innerhalb des Rohrreaktors mit Hilfe von mindestens zwei im Rohrreaktor eingebauten Mischmodulen erfolgt.

**12.** Verfahren nach einem der Ansprüche 10 bis 11, **dadurch gekennzeichnet, dass** der Anteil des Volumens des bzw. der Mischmodule am Gesamtvolumen des Reaktors 20 bis 80 % beträgt.

**13.** Verfahren nach einem der Ansprüche 11 bis 12, **dadurch gekennzeichnet, dass** der Abstand zwischen zwei benachbarten Mischmodulen des 0,2 bis 5fache der Länge der Mischmodule beträgt.

**14.** Verfahren nach einem der Ansprüche 11 bis 13, **dadurch gekennzeichnet, dass** der Abstand zweier benachbarter Mischmodule in Fließrichtung abnimmt.

**15.** Verfahren nach einem der Ansprüche 1 bis 14, **dadurch gekennzeichnet, dass** der Belastungsfaktor im Bereich von 0,2 bis 0,8 liegt.

**Claims**

**1.** Process for continuously preparing $\alpha,\beta$-unsaturated $C_{10}$-aldehydes from aliphatic $C_5$-aldehydes, which comprises the following steps:

a) aldol condensation of aliphatic $C_5$-aldehydes to give $\alpha,\beta$-unsaturated $C_{10}$-aldehydes in the presence of an aqueous base in a tubular reactor;
b) phase separation of the output from the tubular reactor into an aqueous catalyst phase and an organic product phase;
c) separation of the organic product phase into $\alpha,\beta$-unsaturated $C_{10}$-aldehydes, aliphatic $C_5$-aldehydes and by-products;
d) discharge of a portion of the aqueous catalyst phase to remove the water of reaction and supplementation with fresh base and subsequent recycling into the tubular reactor;

**characterized in that**
the aliphatic $C_5$-aldehydes and/or the $\alpha,\beta$-unsaturated $C_{10}$-aldehydes are dispersed as droplets in the aqueous base; wherein the average Sauter diameter of the droplets is in the range from 0.2 mm to 1.54 mm.

**2.** Process according to Claim 1, **characterized in that** the pH of the aqueous base is in the range from 12.5 to 13.5, and the aqueous base comprises sodium hydroxide and sodium salts of carboxylic acids.

**3.** Process according to Claim 1 or 2, **characterized in that** the phase separation of the output from the tubular reactor into the aqueous catalyst phase and the organic product phase is effected at temperatures in the range from 70°C to 120°C.

**4.** Process according to Claim 1, 2 or 3, **characterized in that** the reaction temperature in the tubular reactor is in the range from 120°C to 140°C.

**5.** Process according to any one of Claims 1 to 4, **characterized in that** the ratio of the mass of aqueous base to the mass of the aliphatic $C_5$-aldehydes at the inlet of the tubular reactor is in the range from 5 to 20.

**6.** Process according to any one of Claims 1 to 5, **characterized in that** the average superficial velocity of the mixture of aqueous base and aliphatic $C_5$-aldehydes is in the range from 0.5 to 4 m/s, especially in the range from 1 to 2.5 m/s.

**7.** Process according to any one of Claims 1 to 6, **characterized in that** the average residence time of the starting mixture in the tubular reactor is 40 to 360 seconds, especially 60 to 180 seconds.

**8.** Process according to any one of Claims 1 to 7, **characterized in that** a $C_5$-aldehyde mixture with an n-pentanal content of at least 90% by mass is used.

**9.** Process according to any one of Claims 1 to 8, **characterized in that** the aliphatic $C_5$-aldehydes and/or the $\alpha,\beta$-unsaturated $C_{10}$-aldehydes are dispersed in the aqueous base within the tubular reactor.

**10.** Process according to Claim 9, **characterized in that** the aliphatic $C_5$-aldehydes and/or the $\alpha,\beta$-unsaturated $C_{10}$-aldehydes are dispersed in the aqueous base within the tubular reactor with the aid of at least one mixing module installed in the tubular reactor.

**11.** Process according to Claim 10, **characterized in that** the aliphatic $C_5$-aldehydes and/or the $\alpha,\beta$-unsaturated $C_{10}$-aldehydes are dispersed in the aqueous base within the tubular reactor with the aid of at least two mixing modules installed in the tubular reactor.

**12.** Process according to any one of Claims 10 to 11, **characterized in that** the proportion of the volume of the mixing module(s) in the total volume of the reactor is 20 to 80%.

**13.** Process according to any one of Claims 11 to 12, **characterized in that** the distance between two adjacent mixing modules is 0.2 to 5 times the length of the mixing modules.

**14.** Process according to any one of Claims 11 to 13, **characterized in that** the distance between two adjacent mixing modules decreases in flow direction.

**15.** Process according to any one of Claims 1 to 14, **characterized in that** the loading factor is in the range from 0.2 to 0.8.

**Revendications**

**1.** Procédé de fabrication continue d'aldéhydes en $C_{10}$ $\alpha,\beta$-insaturés à partir d'aldéhydes aliphatiques en $C_5$, qui comprend les étapes suivantes :

a) la condensation aldolique d'aldéhydes aliphatiques en $C_5$ en aldéhydes en $C_{10}$ $\alpha,\beta$-insaturés en présence d'une base aqueuse dans un réacteur tubulaire ;
b) la séparation de phases de la sortie du réacteur tubulaire en une phase aqueuse de catalyseur et une phase organique de produits ;
c) la séparation de la phase organique de produits en aldéhydes en $C_{10}$ $\alpha,\beta$-insaturés, aldéhydes aliphatiques en $C_5$ et produits secondaires ;
d) la purge d'une partie de la phase aqueuse de catalyseur pour éliminer l'eau de réaction et le complément avec une lessive fraîche, puis le recyclage dans le réacteur tubulaire ;

**caractérisé en ce que** les aldéhydes aliphatiques en $C_5$ et/ou les aldéhydes en $C_{10}$ $\alpha,\beta$-insaturés sont dispersés sous la forme de gouttes dans la base aqueuse, le diamètre de Sauter moyen des gouttes étant compris entre 0,2 mm et 1,54 mm.

**2.** Procédé selon la revendication 1, **caractérisé en ce que** le pH de la base aqueuse se situe dans la plage allant de 12,5 à 13,5, et **en ce que** la base aqueuse contient de l'hydroxyde de sodium, ainsi que des sels de sodium d'acides carboxyliques.

**3.** Procédé selon la revendication 1 ou 2, **caractérisé en ce que** la séparation de phases de la sortie du réacteur tubulaire en la phase aqueuse de catalyseur et la phase organique de produits a lieu à des températures comprises entre 70 °C et 120 °C.

4. Procédé selon la revendication 1, 2 ou 3, **caractérisé en ce que** la température de réaction dans le réacteur tubulaire se situe dans la plage allant de 120 °C à 140 °C.

5. Procédé selon l'une quelconque des revendications 1 à 4, **caractérisé en ce que** le rapport entre la masse de la base aqueuse et la masse des aldéhydes aliphatiques en $C_5$ à l'entrée du réacteur tubulaire se situe dans la plage allant de 5 à 20.

6. Procédé selon l'une quelconque des revendications 1 à 5, **caractérisé en ce que** la vitesse à vide moyenne du mélange de la base aqueuse et des aldéhydes aliphatiques en $C_5$ se situe dans la plage allant de 0,5 à 4 m/s, notamment dans la plage allant de 1 à 2,5 m/s.

7. Procédé selon l'une quelconque des revendications 1 à 6, **caractérisé en ce que** le temps de séjour moyen du mélange d'entrée dans le réacteur tubulaire est de 40 à 360 secondes, notamment de 60 à 180 secondes.

8. Procédé selon l'une quelconque des revendications 1 à 7, **caractérisé en ce qu'**un mélange d'aldéhydes en $C_5$ ayant une teneur en n-pentanal d'au moins 90 % en masse est utilisé.

9. Procédé selon l'une quelconque des revendications 1 à 8, **caractérisé en ce que** la dispersion des aldéhydes aliphatiques en $C_5$ et/ou des aldéhydes en $C_{10}$ $\alpha,\beta$-insaturés dans la base aqueuse a lieu dans le réacteur tubulaire.

10. Procédé selon la revendication 9, **caractérisé en ce que** la dispersion des aldéhydes aliphatiques en $C_5$ et/ou des aldéhydes en $C_{10}$ $\alpha,\beta$-insaturés dans la base aqueuse a lieu dans le réacteur tubulaire à l'aide d'au moins un module de mélange installé dans le réacteur tubulaire.

11. Procédé selon la revendication 10, **caractérisé en ce que** la dispersion des aldéhydes aliphatiques en $C_5$ et/ou des aldéhydes en $C_{10}$ $\alpha,\beta$-insaturés dans la base aqueuse a lieu dans le réacteur tubulaire au moyen d'au moins deux modules de mélange installés dans le réacteur tubulaire.

12. Procédé selon l'une quelconque des revendications 10 à 11, **caractérisé en ce que** la proportion du volume du ou des modules de mélange par rapport au volume total du réacteur est de 20 à 80 %.

13. Procédé selon l'une quelconque des revendications 11 à 12, **caractérisé en ce que** la distance entre deux modules de mélange voisins est de 0,2 à 5 fois la longueur des modules de mélange.

14. Procédé selon l'une quelconque des revendications 11 à 13, **caractérisé en ce que** la distance entre deux modules de mélange voisins diminue dans la direction d'écoulement.

15. Procédé selon l'une quelconque des revendications 1 à 14, **caractérisé en ce que** le facteur de charge se situe dans la plage allant de 0,2 à 0,8.

Fig. 1

Fig. 2

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19957522
- DE 10108474
- DE 10108475
- DE 10108476
- DE 10225282
- WO 9320034 A

- EP 1103538 A
- DE 102008002187
- DE 19849922
- DE 19849924
- DE 19956410

**In der Beschreibung aufgeführte Nicht-Patentliteratur**

- **COULALOGLOU C.A.** *AICHE Journal,* 1976, vol. 22 (2), 289-295
- **R. K. THAKUR.** *Trans IChemE,* 2003, vol. 81, 787-826
- **STREIFF F.** *Recent Prog.Genie Proc.,* 1997, vol. 11 (51), 307

- **R.K.THAKUR.** *Trans IChemE,* 2003, vol. 81, 787-826
- **F. STREIFF.** *Recents Progres en Genie des Procedes,* 1997, vol. 11 (51
- **M. ZLOKARNIK.** Rührtechnik -Theorie und Praxis. Springer-Verlag, 1999